(19) Europäisches Patentamt / European Patent Office / Office européen des brevets

(11) EP 2 900 648 B1

(12) **FASCICULE DE BREVET EUROPEEN**

(45) Date de publication et mention de la délivrance du brevet:
**17.05.2017 Bulletin 2017/20**

(21) Numéro de dépôt: **13779316.2**

(22) Date de dépôt: **26.09.2013**

(51) Int Cl.:
***C07D 307/12*** *(2006.01)* ***C07D 307/40*** *(2006.01)*

(86) Numéro de dépôt international:
**PCT/FR2013/052272**

(87) Numéro de publication internationale:
**WO 2014/049275 (03.04.2014 Gazette 2014/14)**

(54) **PROCEDE DE SYNTHESE DE 2,5-DI(HYDROXYMETHYL)FURANE ET DE 2,5-DI(HYDROXYMETHYL)TETRAHYDROFURANE PAR HYDROGENATION SELECTIVE DE FURANE-2,5-DIALDEHYDE**

SYNTHETISCHEN VERFAHREN FÜR 2,5-DI(HYDROXYMETHYL)FURAN UND 2,5-DI(HYDROXYMETHYL) TETRAHYDROFURAN DURCH SELECTIV HYDRIERUNG VON FURAN-2,5-DIALDEHYDE

SYNTHETIC PROCESS FOR 2,5-DI(HYDROXYMETHYL)FURAN AND 2,5-DI(HYDROXYMETHYL) TETRAHYDROFURAN VIA SELECTIVE HYDROGENATION OF FURAN-2,5-DIALDEHYDE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **27.09.2012 FR 1259102**

(43) Date de publication de la demande:
**05.08.2015 Bulletin 2015/32**

(73) Titulaire: **Roquette Frères**
**62136 Lestrem (FR)**

(72) Inventeurs:
- **IBERT, Mathias**
  **F-59930 La Chapelle d'Armentieres (FR)**
- **CHAMBON, Flora**
  **F-59700 Marc En Baroeul (FR)**
- **DAMBRINE, Laurent**
  **F-62114 Sains en Gohelle (FR)**

(74) Mandataire: **Cabinet Plasseraud**
**66 rue de la Chaussée d'Antin**
**75440 Paris Cedex 09 (FR)**

(56) Documents cités:
**WO-A1-2007/146836**

- **SCHIAVO V ET AL: "Catalytic hydrogenation of 5-(hydroxymethyl)furfural in aqueous medium", BULLETIN DE LA SOCIETE CHIMIQUE DE FRANCE, SOCIETE FRANCAISE DE CHIMIE. PARIS, FRANCE, 1 janvier 1991 (1991-01-01), pages 704-711, XP008085941, ISSN: 0037-8968 cité dans la demande**
- **YOSHINAO NAKAGAWA ET AL: "Total hydrogenation of furan derivatives over silica-supported Ni Pd alloy catalyst", CATALYSIS COMMUNICATIONS, ELSEVIER SCIENCE, AMSTERDAM, NL, vol. 12, no. 3, 2 septembre 2010 (2010-09-02), pages 154-156, XP028152982, ISSN: 1566-7367, DOI: 10.1016/J.CATCOM.2010.09.003 [extrait le 2010-09-15] cité dans la demande**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 900 648 B1

## Description

La présente invention concerne un procédé d'hydrogénation sélective en 2,5-di(hydroxyméthyl)tétrahydrofurane (DHMTHF). De manière tout à fait avantageuse par rapport à l'art antérieur qui utilise des sucres en C6 ou du 5-hydroxyméthyl furaldéhyde (5-HMF) comme matières premières de départ, le procédé selon la présente invention se déroule à basses températures (préférentiellement entre 30 °C et 120 °C), tout en mettant en oeuvre une faible quantité de catalyseur par rapport au réactif initial (préférentiellement moins de 2 % par rapport au poids du réactif).

Tout en s'affranchissant des contraintes techniques liées à l'emploi de températures élevées, la Demanderesse a donc mis au point un procédé économiquement intéressant en réduisant la quantité de catalyseur mise en oeuvre, autorisant la synthèse sélective de DHMF et de DHMTHF, tout en explorant un nouvelle voie à base de DFF pour atteindre ces composés.

La chimie du furane et de ses dérivés connaît un large essor depuis quelques années. Parmi les composés furaniques d'importance, on peut citer notamment le 2,5-di(hydroxyméthyl)furane (DHMF) et le 2,5-di(hydroxyméthyl)tétrahydrofurane (DHMTHF) qui trouvent des applications dans les adhésifs, les joints, les composites, les revêtements, les mousses, les solvants ou encore comme intermédiaires de synthèse pour la fabrication de polymères, ces applications étant divulguées dans le document US 2011 / 306780. L'utilisation spécifique du DHMF dans la fabrication de mousses polyuréthanes et de polyesters est quant-à-elle relatée dans le document « Biomass into chemicals: Conversion of sugars to furan derivatives by catalytic processes » (Applied Catalysis A: General 285, pp.1-13, 2010).

Le 2,5-di(hydroxyméthyl)furane (DHMF) et le 2,5-di(hydroxyméthyl)tétrahydrofurane (DHMTHF) répondent aux formules suivantes :

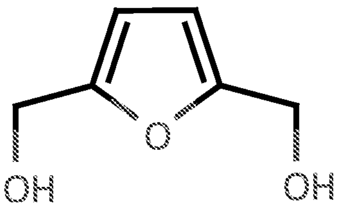

(DHMF)

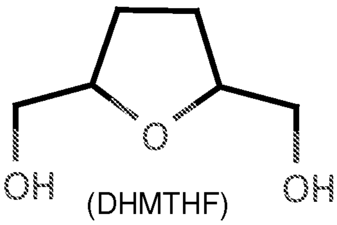

(DHMTHF)

Il existe 2 voies conduisant à l'obtention de ces produits :

- l'hydrogénation catalytique de 5-hydroxyméthyl furaldéhyde (5-HMF) ou
- la catalyse tandem (ou bifonctionnelle) consistant à déshydrater puis hydrogéner des sucres en C6.

Dans la première catégorie, on peut citer le document « Total hydrogenation of furan derivatives over silica-supported Ni-Pd alloy catalyst » (Catal. Comm., 12, pp. 154-156, 2010) qui divulgue la synthèse de DHMTHF à partir de 5-HMF dissous dans de l'eau et du méthanol, en présence d'un catalyseur bimétallique nickel-palladium supporté sur silice. La réaction a lieu pendant 2 heures à 40 degrés, avec un taux de conversion du réactif de 99 % en mole et un rendement en mole de 96 % en DHMTHF. Toutefois, la quantité de catalyseur engagée est importante, car égale à 16 % du poids total de 5-HMF utilisé.

On peut également se référer au document US 7 317 116 qui décrit la synthèse de DHMF en partant du 5-HMF. Ce dernier est dissous dans du méthanol et la réaction est catalysée par du nickel. La conversion est totale et on obtient un rendement supérieur à 90 % en mole de DHMF. Néanmoins, on consomme une quantité non négligeable de catalyseur (6,7 % par rapport au poids de 5-HMF) et on est obligé de travailler à température élevée (150 °C).

Le document WO 2007 / 146836 décrit la synthèse dans l'eau de DHMF, avec un catalyseur massique de type Cobalt de Raney. On travaille alors à 60 °C pour une conversion totale du 5-HMF et un rendement de 97 % en mole de produit final. Il faut souligner qu'on met ici en oeuvre une quantité très importante de catalyseur : celui-ci représente 51 % en poids du 5-HMF de départ.

Enfin, le document "Hydrogenation of 5-hydroxymethylfurfural, in aqueous medium" (Bull. Soc. Chim. Fr., 1991, 128, pp. 704-711) présente la synthèse de DHMF et de DHMTHF avec des rendements respectifs en mole de 100 % et 92 %, avec différents catalyseurs massiques utilisés à raison de 10 % environ par rapport au poids de 5-HMF, et à une température de 140 °C.

L'autre méthode de synthèse de DHMF et de DHMTHF consiste à déshydrater puis hydrogéner par voie catalytique des sucres en C6. A cet égard, on peut citer le document WO 2008 / 053284 qui décrit l'hydrogénation du glucose en présence d'un catalyseur palladium supporté sur charbon, dans une proportion de l'ordre de 3 % par rapport au poids de réactif. Il faut cependant travailler à 130 °C pour obtenir un taux de conversion de 90 % par rapport au glucose et un rendement de 90 % en mole de DHMF.

Dans cette même catégorie, on peut citer enfin le document WO 2012 / 082665 qui se focalise sur le fructose en tant que matière première de départ. Les quantités de catalyseur varient entre 3 et 33 % par rapport au poids de fructose. Aucune information n'est donnée sur les rendements et les taux de conversion ; il apparaît en revanche qu'il faut travailler à 130 °C, voire à 180 °C.

Par conséquent, les procédés de l'art antérieur visant à obtenir du DHMF et / ou du DHMTHF à partir de 5-HMF ou de sucres en C6 présentent l'inconvénient de mettre en oeuvre des températures élevées (nécessité de systèmes de chauffage et d'isolation des installations, de contrôle des conditions de refroidissement, dangerosité pour l'utilisateur) et / ou de consommer des quantités importantes de catalyseur par rapport à la masse de réactif initial (procédés économiquement peu rentables, notamment eu égard au prix du catalyseur).

Travaillant pour pallier ces inconvénients, la Demanderesse est parvenue à mettre au point un nouveau procédé de synthèse de DHMTHF. Ce procédé repose sur l'hydrogénation sélective du DFF, matière première encore jamais envisagée pour la synthèse des composés précités. De manière très avantageuse, ce procédé permet de travailler à des températures relativement peu élevées, notamment préférentiellement comprises entre 30 °C et 120 °C. De plus, les quantités de catalyseur engagées restent très faibles par rapport au DFF initial : elles sont inférieures à 5 % du poids dudit DFF.

Un autre avantage du procédé mis au point par la Demanderesse réside dans la possibilité d'obtenir la formation préférentielle de DHMTHF en fonction des conditions expérimentales. Le réactif de départ est donc une composition contenant du DFF, ce qui signifie que ladite composition peut contenir un autre composé que le DFF. La composition contenant du DFF possède une très haute teneur en DFF ; elle contient ledit DFF en une proportion en poids au moins égale à 90 %, préférentiellement 95 %, très préférentiellement 98 %. De manière tout à fait avantageuse, la composition contenant le DFF dans les proportions susmentionnées est issue de l'oxydation du 5-HMF selon le procédé objet de la Demande de Brevet Français ayant pour numéro de dépôt 1162343. Cette Demande décrit un procédé permettant d'obtenir très facilement une composition à haute teneur en DFF à partir de 5-HMF, le 5-HMF de départ ne présentant pas obligatoirement un grand degré de pureté. Par opposition, les procédés de fabrication de DHMF et de DHMTHF à partir de 5-HMF requièrent en général un très haut degré de pureté en 5-HMF.

Dans la composition initiale de DFF, le reliquat est en général du FFCA (acide 5-furaldéhyde-2-carboxylique) issu du procédé objet de la Demande de Brevet précitée. La composition initiale contient alors de préférence au moins 90 % en poids de DFF, préférentiellement au moins 95 % en poids de DFF, plus préférentiellement 99 % en poids de DFF, le FFCA représentant dans chacun de ces cas le reliquat de la composition.

Aussi, un premier objet de la présente invention consiste en un procédé de synthèse de DHMTHF, par mise en contact :

a) d'une composition contenant du DFF à haute teneur (> 90%),
b) d'un solvant protique,
c) d'une source en hydrogène,
d) et d'un catalyseur d'hydrogénation

tel que décrit à la revendication 1.

Dans toute la présente demande, on utilise les termes de sélectivité (S), de conversion (C) et de rendement (R) en référence aux définitions suivantes :

$$C\ (\%\ \text{molaire}) = ((\text{quantité de DFF transformée}) \times 100)\ /\ \text{quantité de DFF initiale}$$

$$S = (\text{quantité de DHMF ou de DHMTHF formée})\ /\ \text{quantité de DFF transformée}$$

$$R\ (\%\ molaire) = S \times C / 100$$

Le procédé objet de la Demande de Brevet n° 1162343 précitée est caractérisé en ce qu'il comprend une étape d'oxydation de 5-HMF en présence d'au moins un acide organique, un radical nitroxyl, une source en oxygène et un agent de transfert d'oxygène. Le radical nitroxyl est notamment choisi parmi les (2,2,6,6-tetramethylpiperidin-1-yl)oxyl, encore dénommés TEMPO.

Le solvant protique mis en oeuvre dans la présente invention est plus précisément choisi parmi l'eau, le méthanol, l'éthanol, l'isopropanol, le propanol-1, le butanol-1 et les mélanges de ces solvants, l'eau étant le solvant préféré.

La réaction de synthèse par hydrogénation a lieu à une température comprise entre 50 °C et 120 °C.

La source en hydrogène est choisie parmi toutes les sources accessibles à l'homme du métier. Il s'agit de l'hydrogène moléculaire, qui est généralement conservé dans des réceptacles pressurisés. L'hydrogène moléculaire est sous une pression comprise entre 1 bar et 400 bars, préférentiellement entre 10 bars et 110 bars.

Le catalyseur d'hydrogénation est choisi parmi les catalyseurs hétérogènes bien connus de l'homme du métier, et notamment parmi les catalyseurs métalliques classiques d'hydrogénation. Le catalyseur est de type supporté. Le support est le charbon actif. La teneur en métal représente de 0,1 % à 90 % du poids total du catalyseur. Le catalyseur est à base de Pd.

Le temps de contact entre les différents constituants a), b), c) et d) est compris entre 1 minute et 8 heures, préférentiellement entre 15 minutes et 2 heures.

Pour le reste, le procédé d'hydrogénation de la présente invention peut-être réalisé dans tous types de réacteurs batch ou lit fixe sous pression ou non, préférentiellement sous pression. Le type de réacteur batch peut être un autoclave muni d'un agitateur à vitesse variable. Préférentiellement, le réacteur est en outre muni d'un tube d'admission de gaz, connecté à un système sous pression munie d'un détendeur, et d'un tube d'évacuation de gaz. Le réacteur peut en outre comprendre un système de refroidissement et également un système de mesure et de régulation de la température. Dans le cadre d'un réacteur batch, la quantité de catalyseur est comprise entre 0,01 % et 30 %, plus préférentiellement entre 0,1 % et 5 %, très préférentiellement entre 0,15 % et 2,5 % par rapport au poids de la composition de DFF.

Au cours du procédé d'hydrogénation, le milieu réactionnel est maintenu sous agitation. La vitesse d'agitation est préférentiellement réglée entre 800 et 2200 tours / minute, plus préférentiellement entre 1500 et 1700 tours / minute.

Le procédé selon l'invention peut en outre comprendre un étape de filtration du milieu réactionnel issu de l'étape d'hydrogénation permettant ainsi de séparer le ou les produits de réaction DHMF et/ou de DHMTHF du catalyseur solide.

Grâce notamment à la haute teneur en DFF de la composition de départ, on peut aisément recycler le catalyseur utilisé dans la présente invention. La Demanderesse a même pu constater, au cours des expériences qu'elle a menées, que l'activité du catalyseur n'était pas modifiée, même lorsque ledit catalyseur avait été recyclé 5 fois.

Les exemples qui suivent permettent de mieux appréhender la présente invention, sans toutefois en limiter la portée.

## EXEMPLES

Exemple de synthèse de DHMTHF en présence du catalyseur palladium supporté sur charbon

Dans cet essai on met en oeuvre :

- une composition contenant du DFF (93,1 % poids de DFF et 6,9 % en poids de FFCA) : 9 g
- un catalyseur palladium supporté sur charbon Pd/C à raison de 0,2 % en poids de Pd par rapport au poids de DFF
- de l'eau déminéralisée : 420 mL

Conditions opératoires :

- température de réaction : 120 °C
- pH initial de réaction : 5,5
- pression en hydrogène : 50 bars
- durée de réaction : 1 h15

Dans un autoclave en inox d'une capacité interne de 600 ml sont introduits 9 g d'une composition contenant du DFF, et le catalyseur commercial Pd/C ainsi que 420 ml d'eau déminéralisée. Le pH du milieu réactionnel est ajusté à 5,5. Lorsque tous les réactifs sont placés dans l'autoclave, celui-ci est purgé sous azote, puis mis sous 50 bars d'hydrogène au début de la chauffe sous agitation (1600 tours / minute). Dès 70 °C, une consommation en hydrogène

est observée, montrant le début de la réaction d'hydrogénation. Après 1 h15 de temps de contact, l'autoclave est mis en refroidissement à l'aide du serpentin de refroidissement et par coupure de la chauffe. L'agitation est ramenée à 250 tours / minute pendant le refroidissement. Lorsque le réacteur atteint 30°C, l'agitation est arrêtée et l'autoclave est dépressurisé. L'autoclave est ouvert, et le milieu réactionnel obtenu est filtré sur filtre millipore 8 μm pour séparer le milieu réactionnel du catalyseur solide, pouvant être réengagé dans une autre réaction d'hydrogénation. Un échantillon du brut réactionnel est prélevé puis analysé en chromatographie en phase gazeuse.

Une conversion de 100 % est observée avec une sélectivité en DHMTHF de 79 %.

Cet essai correspond à l'essai n° 10 dans le tableau 1.

Exemple de synthèse non conforme à la présente invention de DHMF en présence du catalyseur palladium supporté sur charbon

Dans cet essai on met en oeuvre :

- une composition contenant du DFF (99,2 % en poids de DFF et 0,8 % en poids de FFCA) : 10 g
- un catalyseur palladium supporté sur charbon Pd/C à raison de 0,2 % en poids de Pd par rapport au poids de DFF
- de l'eau déminéralisée : 400 mL

Conditions opératoires :

- température de réaction : 70°C
- pH initial de réaction : 5
- pression en hydrogène : 20 bars
- durée de réaction : 1 h30

Dans un autoclave en inox d'une capacité interne de 600 ml sont introduits 10 g d'une composition contenant du DFF et le catalyseur Pd/C ainsi que 400 ml d'eau déminéralisée. Le pH du milieu réactionnel est ajusté à 5. Lorsque tous les réactifs sont placés dans l'autoclave, celui-ci est purgé sous azote, puis mis sous 20 bars d'hydrogène au début de la chauffe sous agitation (1600 tours / minute). Dès 60°C, une consommation en hydrogène est observée, montrant le début de la réaction d'hydrogénation. Après 1 h30 de temps de contact, l'autoclave est mis en refroidissement à l'aide du serpentin de refroidissement et par coupure de la chauffe. L'agitation est ramenée à 250 tours / minute pendant le refroidissement. Lorsque le réacteur atteint 30°C, l'agitation est arrêtée et l'autoclave est dépressurisé. L'autoclave est ouvert, et le milieu réactionnel obtenu est filtré sur filtre millipore 8 μm pour séparer le milieu réactionnel du catalyseur solide, celui-ci pouvant être réengagé dans une autre réaction d'hydrogénation.

Un échantillon du brut réactionnel est prélevé puis analysé en chromatographie en phase gazeuse. Une conversion de 100 % est observée avec une sélectivité en DHMF de 83,2 %. Cet essai correspond à l'essai n° 12 dans le tableau 1.

Exemple de synthèse non conforme à la présente invention de DHMF en présence du catalyseur massique Nickel de Raney :

Dans cet essai on met en oeuvre :

- une composition contenant du DFF (97.4 % poids de DFF et 2.6 % en poids de FFCA) : 10g
- un catalyseur massique Nickel de Raney à raison de 2% en poids de Ni par rapport au poids de DFF
- de l'eau déminéralisée : 400 ml

Conditions opératoires :

- température de réaction : 100 °C
- pression en hydrogène : 50 bars
- pH initial de réaction : 8,2
- durée de réaction : 1h00

Dans un autoclave inox d'une capacité interne de 600 ml est introduit 10g d'une composition contenant du DFF, le catalyseur Nickel de Raney ainsi que 400 ml d'eau déminéralisée. Le pH est ajusté à 8,2. Lorsque les réactifs sont placés dans l'autoclave, celui-ci est purgé sous azote, puis mis sous 20 bars d'hydrogène au début de la chauffe sous agitation (1600 tours/minute). Dès 60 °C, une consommation en hydrogène est observée, montrant le début de la

réaction d'hydrogénation. Après 1h00 de temps de contact, l'autoclave est mis en refroidissement à l'aide du serpentin de refroidissement et par coupure de la chauffe. L'agitation est ramenée à 250 tours/minute pendant le refroidissement. Lorsque le réacteur atteint 30 °C, l'agitation est arrêtée et l'autoclave dépressurisé. L'autoclave est ouvert, et le milieu réactionnel obtenu est filtré sur filtre millipore 8 μm pour séparer le milieu réactionnel du catalyseur solide, celui-ci pouvant être réengagé dans une autre réaction d'hydrogénation.

Un échantillon du brut réactionnel est prélevé puis analysé en chromatographie en phase gazeuse. Une conversion de 100 % est observée avec une sélectivité en DHMF de 60,1 %. Cet essai correspond à l'essai n °6 dans le tableau 1.

**Tableau 1**

| n° essai | Composition contenant du DFF | | | Solvant | Catalyseur | | $PH_2$ (bars) | T (°C) | Durée | Conversion (%) | DHMF (%) | DHMTHF (%) |
|---|---|---|---|---|---|---|---|---|---|---|---|---|
| | Teneur en DFF (%) | Teneur en FFCA (%) | Masse (g) | | Nature | % pds métal/ substrat | | | | | | |
| 1 | 93,1 | 6,9 | 10 | $H_2O$ | Ni Raney | 2 | 50 | 95 | 1h00 | 100 | 39 | 25,9 |
| 2 | 99,2 | 0,8 | 10 | $H_2O$ | Ni Raney | 2 | 10 | 65 | 1h30 | 100 | 46,8 | 2,7 |
| 3 | 99,2 | 0,8 | 4,5 | $H_2O$ | Ni Raney | 2 | 50 | 60 | 1h00 | 100 | 34 | 30 |
| 4 | 99,2 | 0,8 | 4,5 | $H_2O$ | Ni Raney | 2 | 100 | 60 | 1h00 | 100 | 55,2 | 14,8 |
| 5 | 97,4 | 2,6 | 30 | $H_2O$ | Ni Raney | 1 | 100 | 50 | 1h15 | 100 | 42,8 | 0,4 |
| 6 | 97,4 | 2,6 | 10 | $H_2O$ | Ni Raney | 2 | 100 | 50 | 1h00 | 100 | 60,1 | 18,2 |
| 7 | 97,4 | 2,6 | 10 | MeOH | Ni Raney | 2 | 100 | 60 | 1h00 | 100 | 43,4 | 0,5 |
| 8 | 97,4 | 2,6 | 10 | EtOH | Ni Raney | 2 | 100 | 60 | 1h00 | 100 | 19,7 | 0,7 |
| 9 | 99,2 | 0,8 | 20 | $H_2O$ | Ni Raney | 2 | 100 | 60 | 1h30 | 100 | 35 | 28,7 |
| 10 | 93,1 | 6,9 | 9 | $H_2O$ | 5% Pd/C | 0,2 | 50 | 120 | 1h15 | 100 | 0 | 78,9 |
| 11 | 97,4 | 2,6 | 10 | $H_2O$ | 5% Pd/C | 0,2 | 20 | 65 | 0h15 | 100 | 0 | 69,4 |
| 12 | 99,2 | 0,8 | 10 | $H_2O$ | 5% Pd/C | 0,2 | 20 | 70 | 1h10 | 100 | 83,2 | 4,9 |
| 13 | 97,4 | 2,6 | 10 | $H_2O$ | 5% Pd/C | 0,2 | 20 | 65 | 0h15 | 100 | 0 | 73,4 |
| 14 | 97,4 | 2,6 | 9 | MeOH | 5% Pd/C | 0,2 | 20 | 45 | 0h15 | 100 | 0 | 70,6 |

(suite)

| n° essai | Composition contenant du DFF | | | Solvant | Catalyseur | | $PH_2$ (bars) | T (°C) | Durée | Conversion (%) | DHMF (%) | DHMTHF (%) |
|---|---|---|---|---|---|---|---|---|---|---|---|---|
| | Teneur en DFF (%) | Teneur en FFCA (%) | Masse (g) | | Nature | % pds métal/ substrat | | | | | | |
| 15 | 97,4 | 2,6 | 9 | EtOH | 5% Pd/C | 0,2 | 20 | 45 | 0h20 | 100 | 0 | 74,7 |

L'ensemble des autres essais n° 1 à 8, 10 et 12 à 14 ont été réalisés de la même manière que décrit précédemment, tout en faisant varier la nature et la quantité de catalyseur, le solvant, le pH, la température, la pression en hydrogène moléculaire et le temps de réaction. Tous les résultats obtenus apparaissent dans le tableau 1.

Ce tableau démontre tout l'intérêt du procédé objet de la présente invention : on peut ainsi, travaillant à faibles températures et sous des pressions modérées, avec de faibles doses de catalyseurs, obtenir de manière sélective du DHMF et du DHMTHF.

## Revendications

1. Procédé de synthèse de 2,5-di(hydroxyméthyl)tetrahydrofurane (DHMTHF), par mise en contact :

   a) d'une composition contenant du furane-2,5-dialdéhyde (DFF),
   b) d'un solvant protique,
   c) d'une source en hydrogène,
   d) et d'un catalyseur d'hydrogénation.

   **caractérisé en ce que** :

   la composition a) possède une teneur en DFF en poids au moins égale à 90 %, préférentiellement 95 %, très préférentiellement 98 %,
   la réaction de synthèse par hydrogénation a lieu à une température comprise entre 50 °C et 120 °C.
   la source en hydrogène c) est de l'hydrogène moléculaire, sous une pression comprise entre 10 bars et 110 bars et,
   le catalyseur d'hydrogénation est à base Pd supporté sur charbon et **en ce que** la teneur en métal représente de 0,1 % à 90 % du poids total du catalyseur.

2. Procédé selon la revendication1, **caractérisé en ce que** la composition a) est issue de l'oxydation du 5-hydroxyméthyl furaldéhyde (5-HMF) en présence d'au moins un acide organique, un radical nitroxyl, une source en oxygène et un agent de transfert d'oxygène.

3. Procédé selon la revendication 1 ou 2, **caractérisé en ce que** solvant protique b) est choisi parmi l'eau, le méthanol, l'éthanol, l'isopropanol, le propanol-1, le butanol-1 et les mélanges de ces solvants, l'eau étant préférée.

4. Procédé selon l'une quelconque des revendications 1 à 3, **caractérisé en ce que** temps de contact entre les différents constituants a), b), c) et d) est compris entre 1 minute et 8 heures, préférentiellement entre 15 minutes et 2 heures.

5. Procédé selon l'une quelconque des revendications 1 à 4, **caractérisé en ce que** le procédé est réalisé dans un réacteur batch ou lit fixe sous pression ou non, préférentiellement sous pression.

6. Procédé selon la revendication 5, **caractérisé en ce que** la quantité de catalyseur est comprise entre 0,01 % et 30 %, plus préférentiellement entre 0,1 % et 5 %, plus préférentiellement entre 0,15 % et 2,5 % par rapport au poids de DFF, dans le cas d'un réacteur batch.

## Patentansprüche

1. Syntheseverfahren für 2,5-Di(hydroxymethyl)tetrahydrofuran (DHMTHF) durch in Kontakt bringen :

   a) einer Zusammensetzung, welche Furan-2,5-dialdehyd (DFF) umfasst,
   b) eines protischen Lösungsmittels,
   c) einer Wasserstoffquelle,
   d) und eines Hydrierungskatalysators,

   **dadurch gekennzeichnet, dass**:

   die Zusammensetzung a) einen DFF-Gewichtsgehalt von mindestens 90 %, bevorzugt 95 %, sehr stark bevorzugt 98 %, aufweist,
die Synthesereaktion durch Hydrierung bei einer Temperatur zwischen 50 °C und 120 °C stattfindet,
es sich bei der Wasserstoffquelle c) um molekularen Wasserstoff mit einem Druck zwischen 10 bar und 110 bar handelt, und
der Hydrierungskatalysator auf Pd auf Kohleträger basiert und der Metallgehalt 0,1 % bis 90 % des Gesamtgewichts des Katalysators ausmacht.

2. Verfahren nach Anspruch 1, **dadurch gekennzeichnet, dass** die Zusammensetzung a) aus der Oxidation von 5-Hydroxymethyl-Furaldehyd (5-HMF) in Anwesenheit wenigstens einer organischen Säure, eines Nitroxylradikals, einer Sauerstoffquelle und eines Sauerstoff-Transfermittels entstanden ist.

3. Verfahren nach Anspruch 1 oder 2, **dadurch gekennzeichnet, dass** das protische Lösungsmittel b) gewählt ist aus Wasser, Methanol, Ethanol, Isopropanol, 1-Propanol, 1-Butanol und Mischungen dieser Lösungsmittel, wobei Wasser bevorzugt ist.

4. Verfahren nach einem der Ansprüche 1 bis 3, **dadurch gekennzeichnet, dass** die Kontaktzeit der verschiedenen Bestandteile a), b), c) und d) zwischen 1 Minute und 8 Stunden liegt, bevorzugt zwischen 15 Minuten und 2 Stunden.

5. Verfahren nach einem der Ansprüche 1 bis 4, **dadurch gekennzeichnet, dass** das Verfahren in einem Batch-Reaktor oder einem Festbett unter Druck oder einem drucklosem Festbett, jedoch bevorzugt unter Druck, durchgeführt wird.

6. Verfahren nach Anspruch 5, **dadurch gekennzeichnet, dass** die KatalysatorMenge im Falle eines Batch-Reaktors zwischen 0,01 % und 30 %, stärker bevorzugt zwischen 0,1 % und 5 %, stärker bevorzugt zwischen 0,15 % und 2,5 %, bezüglich des DFF-Gewichts liegt.

**Claims**

1. A process for synthesizing 2,5-di(hydroxymethyl)furan (DHMF) and/or 2,5-di(hydroxymethyl)tetrahydrofuran DHMTHF, by bringing into contact:

   a) a composition containing furan-2,5-dialdehyde (DFF),
   b) a protic solvent,
   c) a source of hydrogen,
   d) and a hydrogenation catalyst.

   Wherein
   the composition a) has a content of DFF by weight at least equal to 90%, preferably 95%, very preferably 98%,
   the synthesis reaction via hydrogenation takes place at a temperature between 50 °C and
   120 °C,
   the source of hydrogen c) is molecular hydrogen, under a pressure of between 10 bar and 110 bar,
   the hydrogenation catalyst is based on Pd supported on activated carbon and in that the metal content represents from 0.1 % to 90% of the total weight of the catalyst.

2. The process as claimed in claim 1, **characterized in that** the composition a) is derived from the oxidation of 5-hydroxymethyl furaldehyde (5-HMF) in the presence of at least an organic acid, a nitroxyl radical, a source of oxygen and an oxygen transfer agent.

3. The process as claimed in either claim 1 or 2, **characterized in that** the protic solvent b) is selected from water, methanol, ethanol, isopropanol, 1-propanol, 1-butanol and the mixtures of these solvents, water being preferred.

4. The process as claimed in any one of claims 1 to 3, **characterized in that** the contact time between the various constituents a), b), c) and d) is between 1 minute and 8 hours, preferably between 15 minutes and 2 hours.

5. The process as claimed in any one of claims 1 to 4, **characterized in that** the process is carried out in a fixed bed or batch reactor that is pressurized or unpressurized, preferably pressurized.

6. The process as claimed in claim 5, **characterized in that** the amount of catalyst is between 0.01% and 30%, more preferably between 0.1% and 5%, more preferably between 0.15% and 2.5% with respect to the weight of DFF, in the case of a batch reactor.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 2011306780 A
- US 7317116 B
- WO 2007146836 A
- WO 2008053284 A
- WO 2012082665 A
- FR 1162343
- WO 1162343 A


### Littérature non-brevet citée dans la description


- Biomass into chemicals: Conversion of sugars to furan derivatives by catalytic processes. *Applied Catalysis A: General,* 2010, vol. 285, 1-13
- Total hydrogenation of furan derivatives over silica-supported Ni-Pd alloy catalyst. *Catal. Comm.,* 2010, vol. 12, 154-156
- Hydrogenation of 5-hydroxymethylfurfural, in aqueous medium. *Bull. Soc. Chim. Fr.,* 1991, vol. 128, 704-711